# EUROPEAN PATENT SPECIFICATION

(11) **EP 1 743 650 B1**
(45) Date of publication and mention of the grant of the patent: **25.03.2009**
(21) Application number: 06018590.7
(22) Date of filing: 30.10.1997
(51) Int. Cl.: A61K 38/21, A61P 17/00

(54) **Feline intrerferon against feline atopic dermatitis**
Felines Interferon gegen Feline atopische Dermatitis
Interféron félin contre dermatite atopique féline

(30) Priority: 31.10.1996 JP 29060196
(43) Date of publication of application: 17.01.2007
(62) Divisional of application: 97909701.1
(73) Proprietor: TORAY INDUSTRIES, INC., Tokyo 103-8666 (JP)
(72) Inventor: Kajimoto, Tsunesuke, Kanagawa 248-0032 (JP); Suzuki, Makoto, Nagoya-shi Aichi 464 (JP); Go, Ryougai, Ikeda-shi Osaka 563 (JP)
(74) Representative: Kador & Partner

(56) References cited:
- EP-A1- 0 414 355
- HELTON-RHODES, K.: "Feline Immunomodulators - Current Veterinary Therapy XII" 1995, BONAGURA, J.D. , PHILADELPHIA , XP009076995 * page 584, column 2, paragraph 1 *
- ADOLF G R ET AL: "PURIFICATION AND CHARACTERIZATION OF NATURAL HUMAN INTERFERON OMEGA 1 TWO ALTERNATIVE CLEAVAGE SITES FOR THE SIGNAL PEPTIDASE" JOURNAL OF BIOLOGICAL CHEMISTRY, AMERICAN SOCIETY OF BIOLOCHEMICAL BIOLOGISTS, BIRMINGHAM,, US, vol. 265, no. 16, 1990, pages 9290-9295, XP002929489 ISSN: 0021-9258
- DOLEN JALE G ET AL: "Undetectable interferon-alpha serum levels in a patient with atopic dermatitis" JOURNAL OF INTERFERON AND CYTOKINE RESEARCH, vol. 15, no. 11, 1995, pages 973-975, XP009076981 ISSN: 1079-9907
- HALPERN MICHAEL ET AL: "Regulation of the low affinity IgE Fc receptor (CD23) in atopic dermatitis" INTERNATIONAL ARCHIVES OF ALLERGY AND IMMUNOLOGY, vol. 100, no. 3, 1993, pages 197-200, XP009076980 ISSN: 1018-2438
- SCOTT D W ET AL: "Medical management of allergic pruritus in the cat, with emphasis on feline atopy." JOURNAL OF THE SOUTH AFRICAN VETERINARY ASSOCIATION. JUN 1993, vol. 64, no. 2, June 1993 (1993-06), pages 103-108, XP009076899 ISSN: 1019-9128

## Description

### Technical field

The present invention relates to a therapeutic agent for feline atopic dermatitis.

### Prior Arts

As a feline interferon, a genetically recombinant type ω-interferon preparation is already approved as a therapeutic agent for calicivirus infections, and is marketed under a trade name of "INTERCAT" since February, 1994.

Presently known interferons include alpha (α) interferons, beta (β) interferon, gamma(γ) interferon, omega (ω) interferon and tau (τ) interferon. As human interferons, three types of α, β and γ are practically applied, and as a feline interferon, an ω-interferon only is practically applied. "INTERCAT" is a genetically recombinant type ω-feline interferon preparation, and It is an injection preparation obtained by infecting Bombyx mori with a baculovirus recombined with the gene of an ω-feline interferon, producing the interferon in the body, extracting and purifying it, adding gelatin and D-sorbitol as a stabilizer and recipient, and freeze-drying the mixture. The genetically recombinant type ω-feline interferon is a glycoprotein with a molecular weight of about 25000, and its protein portion has the amino acid sequence as shown in sequence 1 of the sequence table.

The ω-feline interferon can also be produced by other methods than the Bombyx mori method. For example, it can be produced by transient expression methods using animal cells such as simian COS cells and gene recombination techniques using Chinese hamster's CHO cells, Escherichia coli, yeast, trans-genic animals, etc.

As for the usage and dose of "INTERCAT" approved as a therapeutic agent for calicivirus infections, it is specified to administer 2.5 ~5 MU/kg of feline interferon intravenously three times every other day. In this case, MU (mega unit) is a method for expressing a titer with the antiviral activity of an interferon as an indicator, and expresses one million units.

For feline atopic dermatitis, there is no satisfactory therapeutic method even for human atopic dermatitis, and symptomatic therapeutic methods using steroid hormone preparations are frequently adopted. Steroid hormone preparations have side effects, and the symptomatic therapeutic methods are insufficient in therapeutic effect. Feline atopic dermatitis is an intractable disease. Generally observed feline allergic dermatitis is mostly atopic dermatitis including miliary eczema rotating to parasites such as fleas and eosinophilic granuloma syndrome. Hitherto, drugs such as prednisolone and amcinolone are said to be effective, and they tend to be used more frequently. However, these drugs have a problem of side effects.

It was reported in an American medical magazine in 1990 (M. Boouniewwicz et al., American J. Medicine. 8.8, 365-370 (1990)) that a human γ (gamma) interferon is effective for human atopic dermatitis.

However, this method is not sufficient in the effect of treating feline atopic dermatitis since human interferons are different in action from feline Interferons.

K. Helton Rhodes reports in "Dermatologic Diseases", p. 581- 584 (1995) on feline immunomodulators such as interferon-α-2b.

A task of the present invention is to provide a new excellent therapeutic agent for feline atopic dermatitis.

### Disclosure of the Invention

It has been found that a therapeutic agent containing an ω-feline Interferon is a new excellent therapeutic agent for feline atopic stomatitis.

The task of the present invention has been industrially advantageously achieved by the present invention with the following constitution.

A therapeutic agent for use in the treatment of feline atopic dermatitis, ω-feline interferon is the active agent.

A therapeutic agent for feline atopic dermatitis, as described above, wherein the ω-feline interferon is a a genetically recombinant type interferon.

A therapeutic agent for feline atopic dermatitis, as described above, wherein the ω-feline interferon is an interferon combined with a sugar chain having the amino acid sequence shown by sequence number: 1.

A therapeutic method for feline atopic dermatitis, wherein the therapeutic agent for atopic dermatitis stated above is injected into a cat.

A therapeutic agent for feline atopic dermatitis, as described above, wherein the injection is subcutaneous injection.

A therapeutic agent for feline atopic dermatitis, as described above, wherein the dose is 0.1 -5 MU/kg.

### The Best Embodiments of the Invention

The feline interferon used in the present invention is an ω-feline interferon which can be as produced naturally or can be synthetically synthesized or by any gene recombination technique.

For example, an ω-feline interferon produced by a gene recombination technique and marketed under a trade name of "INTERCAT" (produced by Toray Industries, Inc.) can be used.

The "INTERCAT" has been approved and practically applied as a therapeutic agent for feline calicivirus infections, and mainly contains an interferon combined with a sugar chain having the sequence of 170 amino acids shown in sequence number: 1, and it is obtained by Infecting larvae of Bombyx mori with a recombinant baculovirus, i.e., an insect virus recombined with the gene of an ω-feline interferon, and extracting, separating and refining the interferon produced in the bodies of Bombyx mori.

However, the ω-feline interferon of the present invention is not necessarily limited to said genetically recombinant type feline interferon.

The ω-feline interferons produced by gene manipulation using Escherichia coli. Bacillus subtills and animal cells such as CHO and also the ω-interferon produced from feline cells can also be used. However, in the present situation, the ω feline interferon produced from Bombyx mori is available at low cost.

Feline atopic dermatitis is described below. The therapeutic agent for feline atopic dermatitis of the present invention is a preparation containing an ω-feline Interferon as a principal agent, and when it is used, a solution obtained by dissolving it into physiologic salt solution or infusion solution or any other solution is injected. The injection route can be subcutaneous, intravenous or intramuscular. Subcutaneous injection is preferably simple and practical. The number of administration times is not especially limited, but it is practical to administer once a day every day or 1 to 3 times per week. The dose is not limited either, but is usually 0.1 to 5 MU/kg. A preferable range is 1 to 2.5 MU/kg. The administration effect can be clearly observed from about the 2nd week in most cases.

The ω-feline interferons usually do not cause remarkable fever after administration unlike human beings, and even if fever occurs, the body temperature rise as slight as about 1°C only occurs for a while. Serious side effects such as vomition and diarrhea are not caused.

### Examples

The present invention is described below with reference to examples, but is not limited thereto or thereby. The blood cell count is in number per microliter (/µl).

### [Example 1]

A 4-year-old unsexed female house cat (short hair, Japanese cat) (white, body weight 2.62 kg) came to hospital for the main reason that red eczema occurred in the abdominal part since several days before, and was diagnosed to suffer atopic dermatitis. A preparation containing a ω feline interferon (recombinant type) as a principal agent, i.e., "INTERCAT" was dissolved into physiological solution, and the INTERCAT solution was subcutaneously injected by 5 MU/head (1.9 MU/kg). The administration of "INTERCAT" was continued at intervals of twice a week, and after 8 weeks, the eczema perfectly vanished.

### [Example 2]

A 3-year-old female house cat (Cornish Rex) (white, body weight 2.76 kg) had eosinophilic plaques formed with the hypertrophy of the dorsolumber skin since several months before, and was cured temporarily by periodical administration of a steroid hormone preparation. However, after a white, many plaques occurred on the face and the back. On the auricles, portions considered to show the generation of a fungus existed. The disease was diagnoised as atopic dermatitis. A preparation containing a ω-feline interferon (recombinant type) as a principal agent. i.e., "INTERCAT" was dissolved into physiological salt solution, and the INTERCAT solution was subcutaneously injected by 5 MU/head (1.81 MU/kg). An antihistaminic agent used since before was also used together. The administration of "INTERCAT" was continued at Intervals of once a week. From the 2nd week, the reddishness of the eczema began to vanish, and after 3 months, only a trace remained to show almost perfect healing.

### [Example 3]

A 6-year-old unsexed female house cat (short hair, Japanese cat) (blackish tiger color, body weight 4.55 kg) had had miliary eczema on the back due to flea allergy since two years before, and the administration of asteroid hormone preparation and thorough flea extermination could bring about a lesion. However, since about one year before, many eosinophilic plaques were formed in the abdominal part, and through the administration of the steroid hormone preparation showed reaction, the effect gradually diminished. The disease was diagnosed as atopic dermatitis. A preparation containing an ω feline Interferon (recombinant type) as a principal agent, i.e., "INTERCAT" was dissolved Into physiological salt solution, and the INTERCAT solution was subcutaneously injected by 5 MU/head (1.1 MU/kg). The administration of "INTERCAT" continued at intervals of once a week. After 2 weeks, reddishness almost vanished, and still after 2 months, the disease did not recur.

### Industrial Applicability

The therapeutic agent according to the present invention containing an ω-feline interferon is a new excellent therapeutic agent for feline atopic dermatitis. The present invention is highly industrially useful.

## Claims

1. ω-Feline interferon for use as a therapeutic agent against feline atopic dermatitis.

2. ω-Feline interferon according to claim 1, wherein the ω-feline interferon is a genetically recombinant type interferon.

3. ω-Feline interferon according to claim 1 or 2; wherein the ω-feline interferon is administered by a dose of 0.1 MU/kg to 5 MU/kg per cat body weight.

## Patentansprüche

1. ω-Interferon der Katze für die Verwendung als therapeutisches Mittel gegen atopische Dermatitis bei Katzen.

2. ω-Interferon der Katze nach Anspruch 1, wobei das ω-Interferon der Katze Interferon vom Genrekombinationstyp ist.

3. ω-Interferon der Katze nach Anspruch 1 oder 2, wobei das ω-Interferon der Katze mit einer Dosis von 0,1 bis 5 ME/kg pro Körpergewicht der Katze verabreicht wird.

## Revendications

1. Interféron ω félin pour utilisation en tant qu'agent thérapeutique contre la dermatite atopique féline.

2. Interféron ω félin selon la revendication 1, l'interféron ω félin étant un interféron de type recombinant génétique.

3. Interféron ω félin selon la revendication 1 ou 2, l'interféron ω félin étant administré à une dose de 0,1 MU/kg à 5 MU/kg de poids corporel du chat.


### SEQUENCE LISTING

<110> TORAY INDUSTRIES, INC.
<120> Therpeutic agent and method for feline AIDS virus infections and feline atopic dermatitis
<130> K 52 944
<150> WO PCT/JP97/03963
   <151> 1997-10-30
<160> 1
<170> PatentIn version 3.3
<210> 1
   <211> 170
   <212> PRT
   <213> arificial
<400> 1
   Cys Asp Leu Pro Gln Thr His Gly Leu Leu Asn Arg Arg Ala Leu Thr
   1               5                   10                  15
   Leu Leu Gly Gln Met Arg Arg Leu Pro Ala Ser Ser Cys Gln Lys Asp
               20                  25                  30
   Arg Asn Asp Phe Ala Phe Pro Gln Asp Val Phe Gly Gly Asp Gln Ser
           35                  40                  45
   His Lys Ala Gln Ala Leu Ser Val Val His Val Thr Asp Gln Lys Ile
       50                  55                  60
   Phe His Phe Phe Cys Thr Glu Ala Ser Ser Ser Ala Ala Trp Asn Thr
   65                  70                  75                  80
   Thr Leu Leu Glu Glu Phe Cys Thr Gly Leu Asp Arg Gln Leu Thr Arg
                   85                  90                  95
   Leu Glu Ala Cys Val Leu Gln Glu Val Glu Glu Gly Glu Ala Pro Leu
               100                 105                 110
   Thr Asn Glu Asp Ile His Pro Glu Asp Ser Ile Leu Arg Asn Tyr Phe
           115                 120                 125
   Gln Arg Leu Ser Leu Tyr Leu Glu Glu Lys Lys Tyr Ser Pro Cys Ala
       130                 135                 140
   Trp Glu Ile Val Arg Ala Glu Ile Met Arg Ser Leu Tyr Tyr Ser Ser
   145                 150                 155                 160
   Thr Ala Leu Gln Lys Arg Leu Arg Ser Glu
                   165                 170